# EUROPEAN PATENT SPECIFICATION

(11) **EP 1 936 358 B1**
(45) Date of publication and mention of the grant of the patent: **06.05.2015**
(21) Application number: 07024122.9
(22) Date of filing: 12.12.2007
(51) Int. Cl.: G01N 21/15, G01N 21/27, G01N 21/53, G01N 21/85, G01N 21/94, G01N 21/958, F02B 77/08, G01N 33/28

(54) **Oil mist detector**
Ölnebeldetektor
Détecteur de bruine d'huile

(30) Priority: 21.12.2006 JP 2006343790
(43) Date of publication of application: 25.06.2008
(73) Proprietor: Horiba, Ltd., Kyoto-city, Kyoto 601-8510 (JP); DAIHATSU DIESEL MFG. CO., LTD., Osaka 531-0076 (JP)
(72) Inventor: Nomura, Toshiyuki, Kyoto-city Kyoto 601-8510 (JP); Mochizuki, Masaru, Osaka 531-0076 (JP); Chujo, Junya, Osaka 531-0076 (JP)
(74) Representative: Müller Hoffmann & Partner

(56) References cited:
- DE-U1- 8 128 634
- GB-A- 804 541
- GB-A- 2 398 382
- GB-A- 2 408 798
- US-A- 5 028 790
- "Another advance in oil mist detection from Graviner" MOTOR SHIP UK, vol. 62, no. 734, September 1981 (1981-09), page 83, XP009097223 ISSN: 0027-2000

## Description

### BACKGROUND OF THE INVENTION

### Field of the Invention

The present invention relates to a light-scattering type oil mist detector for detecting oil mist generated by lubricant oil heated in an internal combustion engine as a result of bearing overheating or the like, and more specifically to an oil mist detector suitable for use in a marine internal combustion engine.

### Description of the Background Art

The oil mist detector of this type is arranged inside a crankcase of an internal combustion engine, and includes: a casing having an oil mist introduction chamber; and a light emitting device and a light receiving device that are so arranged as to face the outer side of a transparent window provided in the oil mist introduction chamber. This oil mist detector detects oil mist by irradiating light to a predetermined detection region in the oil mist introduction chamber from the light emitting device through the transparent window and receiving, by the light receiving device through the transparent window, scattering light generated from the irradiated light hitting the oil mist present in the detection region.

Japanese Unexamined Patent Publication No. 2005-164408 discloses an oil mist detector prepared for preventing dirt from adhering to the transparent window during operation of an internal combustion engine. The oil mist detector has a casing provided with a dual pipe structure that prevents an oil splash (oil droplets) from reaching the transparent window.

However, it is impossible to completely prevent oil mist smaller than the oil splash (oil droplets) from reaching and adhering to the transparent window. It has been proved that oil mist adhering to the transparent window, in some cases, is repeatedly dried and adheres over a long period of time, and is condensed thereby fogging up the transparent window, which results in a remarkable decrease in the transmittance of the transparent window.

Moreover, even if the level at which the transmittance of the transparent window remarkably decreases has not yet been reached, detecting oil mist with the transparent window being kept dirty poses a problem of occurrence of detection level error.

On the other hand, the oil mist detector with deteriorated sensitivity can be used again after the transparent window is washed, but a conventional oil mist detector has a drawback that it cannot perceive the sensitivity deterioration and thus requires obligatory periodic washing and the like.

GB 2 398 382 A describes an oil mist sensing device of a light scattering type that senses oil mist generated by heat around the time when lubricant is burned and is downsized by making use of characteristics of the light scattering type that is stain-resistant against oil mist.

### SUMMARY OF THE INVENTION

It is a main object of the present invention to easily and reliably detect dirt on a transparent window of an oil mist detector.

The object is solved by an oil mist detector according to claim 1. Further embodiments are defined in the dependent claims.

Specifically, an oil mist detector according to the present invention includes:
a casing having an oil mist introduction chamber; a transparent window provided in the oil mist introduction chamber; and a light emitting device and a light receiving device so arranged as to face an outer side of the transparent window provided in the oil mist introduction chamber. The light emitting device is arranged for irradiating light to a predetermined detection region in the oil mist introduction chamber through the transparent window and the light receiving device is arranged for receiving through the transparent window scattering light generated from the irradiated light hitting oil mist present in the detection region, thereby achieving oil mist detection. A zero point shifting structure is provided for making a given amount of background scattering light from the casing incident on the light receiving device under absence of oil mist in the oil mist introduction chamber. A controller is provided for accepting a detection signal from the light receiving device and performing predetermined calculation processing, wherein the controller comprises a dirt abnormality judgment part which is adapted to receive a zero point signal as a detection signal detecting only background scattering light, and adapted to compare a detection signal value indicated by a detection signal from the light receiving device and a dirt abnormal value being a preset value by which the zero point is allowed to be decreased, and adapted to judge that the transparent window is dirty if the detection signal value is smaller than the dirt abnormal value.

With such configuration, a zero point is intentionally increased by the zero point shifting structure; thus, a decrease in this zero point permits detection of dirt on the transparent window. Therefore, the dirt on the transparent window can be easily and reliably detected.

The zero point shifting structure is provided to minimize the scattering of light emitted by the light emitting device at the inner wall of the casing and therefore to minimize the amount of background scattering light from the casing incident on the light receiving device when no oil mist is present in the oil mist introduction chamber.

Desirably, as a detailed and preferred embodiment of the oil mist detector and a detailed zero point shifting structure in this detector, the casing forms a cylindrical shape, and the zero point shifting structure is formed of light blocking members, and is formed by adjusting a length of a light blocking member so provided as to partition an optical axis extended line of the irradiated light traveling from the transparent window toward the detection region and an optical axis extended line of the scattering light traveling from the detection region toward the transparent window.

To automatically judge dirt on the transparent window, the controller for accepting a detection signal from the light receiving device and performing predetermined calculation processing is provided.

Desirably, to automatically alarm dirt on the transparent window to the operator, an alarm device for receiving a judgment signal from the dirt abnormality judgment part and alarming the dirt on the transparent window is provided.

According to the present invention configured as described above, dirt on the transparent window of the oil mist detector can be easily and reliably detected.

### BRIEF DESCRIPTION OF THE DRAWINGS

FIG. 1 is an overall perspective view showing an entire oil mist detector according to a preferred embodiment of the present invention;
FIG. 2 is a use condition explanatory diagram showing use condition of the oil mist detector of the same preferred embodiment;
FIG. 3 is a schematic horizontal sectional view showing inner structure of an end of the oil mist detector of the same preferred embodiment;
FIG. 4 is a device configuration diagram of a controller of the same embodiment; and
FIG. 5 is a function configuration diagram of the controller of the same preferred embodiment.

### DETAILED DESCRIPTION OF THE PREFERRED EMBODIMENTS

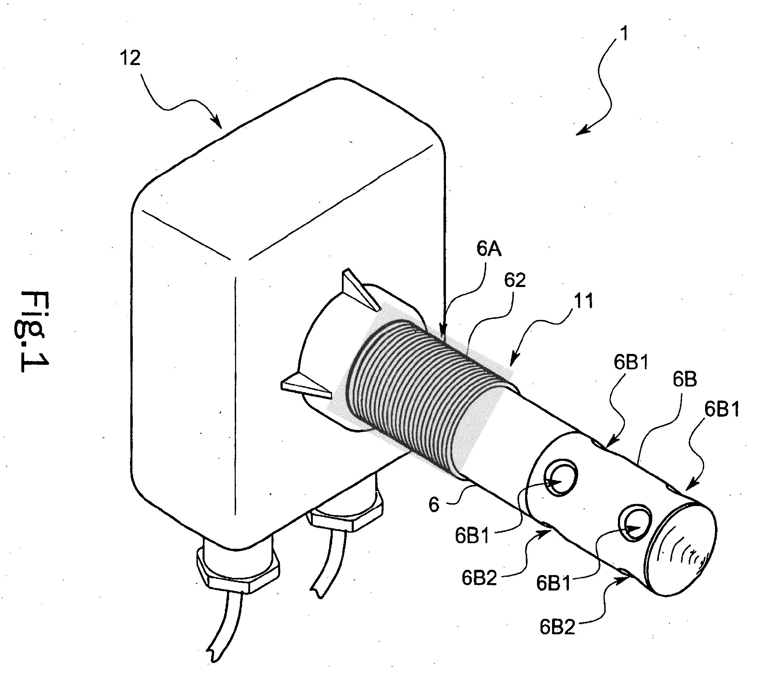

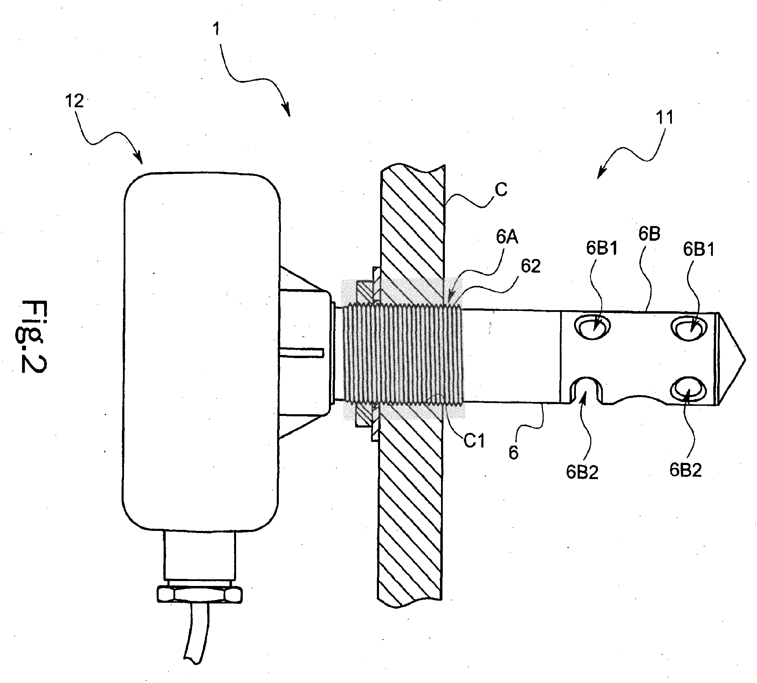

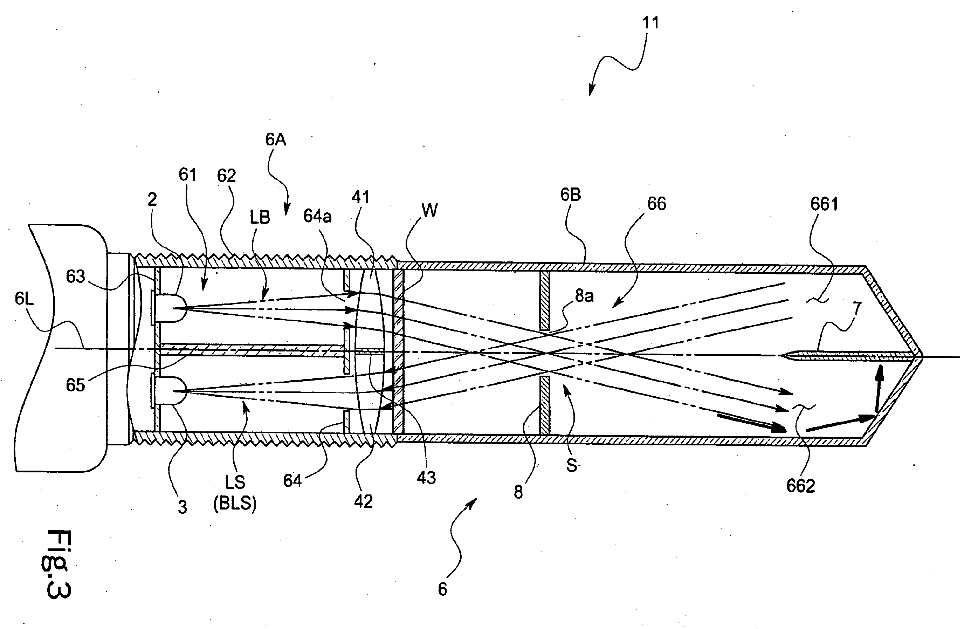

Hereinafter one preferred embodiment of an oil mist detector according to the present invention will be described, referring to the accompanying drawings. FIG. 1 is an overall perspective view showing the entire oil mist detector 1 according to the present preferred embodiment. FIG. 2 is a use condition explanatory diagram showing use condition of this oil mist detector 1. FIG. 3 is a schematic horizontal sectional view showing an inner structure of this oil mist detector 1.

The oil mist detector 1 according to a preferred embodiment of the present invention is, as shown in FIGS. 1 and 2, of a light-scattering type that is fitted to, for example, a crankcase C of a marine diesel engine. The oil mist detector 1 is used for detecting oil mist generated inside the crankcase C as a result of bearing overheating or the like.

### <Device configuration>

Specifically, the oil mist detector 1, as shown in FIGS. 1 and 2, is provided with a detection part 11 of a cylindrical shape and a controller 12 of a rectangular shape. The detection part 11 is so fitted as to penetrate, from the outside, through a fitting hole C1 provided in a wall of the crankcase C with a leading end of the detection part 11 protruding into the crankcase C downward at substantially 0 to approximately 15 degrees. The controller 12 is provided consecutively with the base end of this detection part 11.

The detection part 11 is, as schematically shown in FIG. 3, provided with a casing 6 having: an oil mist introduction chamber 66 formed at the leading end thereof so as to permit introducing and diffusing oil mist inside; and a sensor storage chamber 61 formed at the base end thereof. This oil mist introduction chamber 66 is provided with a transparent window W formed of a transparent plate material, on the outer side of which the sensor storage chamber 61 is disposed. That is, an oil mist introduction chamber 66 and the sensor storage chamber 61 are partitioned by the transparent window W formed of a thin plate-like transparent plate material, such as a film. At the position, in the sensor storage chamber 61, facing the transparent window W (inner side of the base end of the casing 6), a light emitting device 2 and a light receiving device 3 are arranged side by side.

First, the light emitting device 2 and the light receiving device 3 will be described.

The light emitting device 2 is, for example, an LED arranged with a light emitting surface thereof facing the transparent window W, and the present embodiment uses the one which emits light of a wavelength band suitable for a particle size of oil mist. Needless to say, any of other light emitting device 2, such as an LD (laser diode), tungsten lamp, halogen lamp, may be used.

The light receiving device 3 is, for example, a PD (photodiode), with a light receiving surface thereof facing the transparent window W, and outputs an electric signal in accordance with the intensity of light received on the receiving surface. Needless to say, any of other light receiving device 3, such as CCD cadmium sulfide cell and the like, may be used.

These light emitting device 2 and light receiving device 3 are arranged side by side in such a manner that an optical axis of light LB irradiated and an optical axis of light LS received are substantially parallel to each other. As schematically shown in FIG. 3, the light emitting device 2 and the light receiving device 3 are partitioned by a partition plate 65 arranged along a main axis 6L of the casing 6.

The irradiated light LB emanated from the light emitting device 2 is bent toward the light receiving device 3 by the first refraction device 41 arranged between the light emitting surface of the light emitting device 2 and the transparent window W, and then irradiated through the transparent window W to a detection region S set near the casing main axis 6L of the oil mist introduction chamber 66. On the other hand, the scattering light LS generated from the irradiated light LB hitting oil mist present in the detection region S, after passing through the transparent window W, is bent by the second refraction device 42 arranged between the light receiving surface of the light receiving device 3 and the transparent window W and then received by the light receiving device 3.

The first refraction device 41 and the second refraction device 42 respectively form half circles obtained by halving a circular convex lens by a straight line passing through the optical axis (center) of the convex lens, with a thin plate-like first light blocking member 43 sandwiched between the halved surfaces.

In this manner, the scattering light LS received by the light receiving device 3 is converted into a detection signal, i.e., an election signal in accordance with an intensity of this scattering light LS, and transmitted to the controller 12 through a preamplifier circuit or the like, thereby providing a lamp display indicating an oil mist condition.

Next, the casing 6 will be described. The casing 6 forms a substantially cylindrical shape with a leading end surface thereof sealed, and is provided with: a first casing part 6A forming the sensor storage chamber 61; and a second casing part 6B forming the oil mist introduction chamber 66.

The first casing part 6A is provided with: a circumferential wall 62 having on the outer circumference thereof screw heads to be fitted to a predetermined portion, such as the engine crankcase C; a support plate 63 arranged inside near the base end of the circumferential wall 62 orthogonally to the casing main axis 6L; a window plate 64 with an aperture function arranged inside near the leading end of the circumferential wall 62 orthogonally to the casing main axis 6L; and the partition plate 65 arranged between the support plate 63 and the window plate 64 and halving the space therebetween.

The support plate 63 supports the light emitting device 2 and the light receiving device 3 so that the light emitting surface of the light emitting device 2 and the light receiving surface of the light receiving device 3 are oriented perpendicularly to the casing main axis 6L and also are so located as to be symmetrical to each other with respect to the casing main axis 6L with the partition plate 65 therebetween. The window plate 64 is provided with light-transmissive apertures 64a at positions respectively corresponding to the light emitting surface and the light receiving surface.

The partition plate 65 separates the space of the sensor storage chamber 61 surrounded by the circumferential wall 62, the support plate 63, and the window plate 64 into two symmetrical storage spaces for storing the light emitting device 2 and the light receiving device 3, respectively. The partition plate 65 is so provided as to extend along the casing main axis 6L.

The second casing part 6B forms a substantially cylindrical shape of a diameter substantially equal to that of the first casing part 6A, with the leading end surface of the second casing part 6B sealed. The second casing part 6B is provided with: as shown in FIGS. 1 and 2, oil mist introduction ports 6B2 for introducing oil mist to the inside and oil mist exhaust ports 6B1 for exhausting oil mist to the outside. These oil mist introduction ports 6B2 and the oil mist exhaust ports 6B1 are provided, for example, near a detection region S formed inside the second casing part 6B.

A second light blocking member 7 is provided, in front of the detection region S inside this second casing part 6B, that is, on a side opposite to the refraction device, plus between an optical axis extended line of irradiated light LB traveling from the first refraction device 41 toward the detection region S and an optical axis extended line of reflected and scattering light LS traveling from the detection region S toward the second refraction device 42. This second light blocking member 7 forms a plate-like shape, and is so provided as to extend across between the front of the detection region S and the inner surface of the leading end of the second casing part 6B. The second light blocking member 7 forms two spaces 661 and 662 by halving the leading end side of the second casing part 6B vertically substantially along the main axis 6L. The leading end of the second light blocking member 7 has a knife-edge shape so as to reduce scattering light.

Further, the inner surface of the leading end part of the second casing part 6B is tapered such that its sectional area gradually becomes smaller toward the leading end, and the spaces 661 and 662 divided by the second light blocking member 7 function as black body cavities for absorbing light introduced inside by internally reflecting it a plurality of numbers of time.

Further, at a portion corresponding to the detection regions S inside this second casing part 6B, a third light blocking member 8 is provided orthogonally to the casing main axis 6L. This light blocking member 8 has, for example, an oval-shaped slit 8a formed at the center thereof, and the detection region S is formed in front of and behind the slit 8a including this slit 8a. This slit 8a is formed allowing for preventing the generation of scattering light LS from the irradiated light LB directly hitting the third light blocking member 8.

Thus, the oil mist detector 1 of the present preferred embodiment is provided with a zero point shifting structure for shifting a zero point of a detection signal by making a given amount of background scattering light BLS from an inner wall of the casing 6 incident on the light receiving device 3, under the absence of oil mist in the oil mist introduction chamber 66 (detection region S).

This zero point shifting structure is formed of the second light blocking member 7 and the third light blocking member 8. That is, the zero point shifting structure is formed by providing a short length of the second light blocking member 7 along the casing main axis 6L and a low height of the third light blocking member 8 from the side wall of the second casing part 6B.

More specifically, referring to the second light blocking member 7, the length thereof from the inner surface of the leading end of the second casing part 6B is short. Referring to the third light blocking member 8, an opening of the slit 8a provided in the third light blocking member 8 is formed large. Under the absence of oil mist in the oil mist introduction chamber 66, that is, when the diesel engine is stopped, the background scattering light BLS generated by scattering the irradiated light LB from the light emitting device 2 by the inner wall of the casing 6 is received by the light receiving device 3.

For example, the background scattering light BLS received by the light receiving device 3 is adapted to account for several percentages (for example, 2 to 10%) of a detection full scale (permitted detection values) of the light receiving device 3. The second light blocking member 7 and the third light blocking member 8 are fabricated by, for example, injection molding. In the injection molding, the surface condition of the inner wall of this casing is adjusted by the gloss so that the background scattering light does not vary between a condition where no oil is adhering to the surface and a condition where the surface is wet with oil.

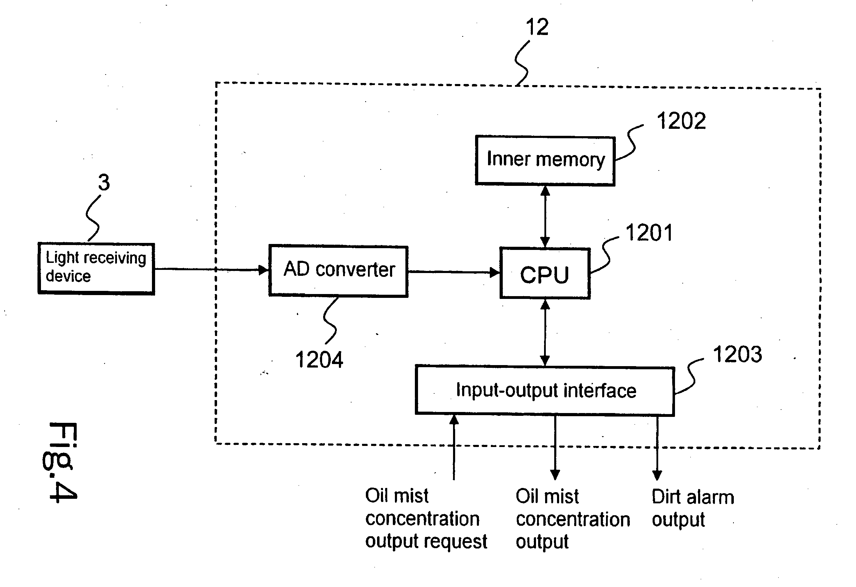

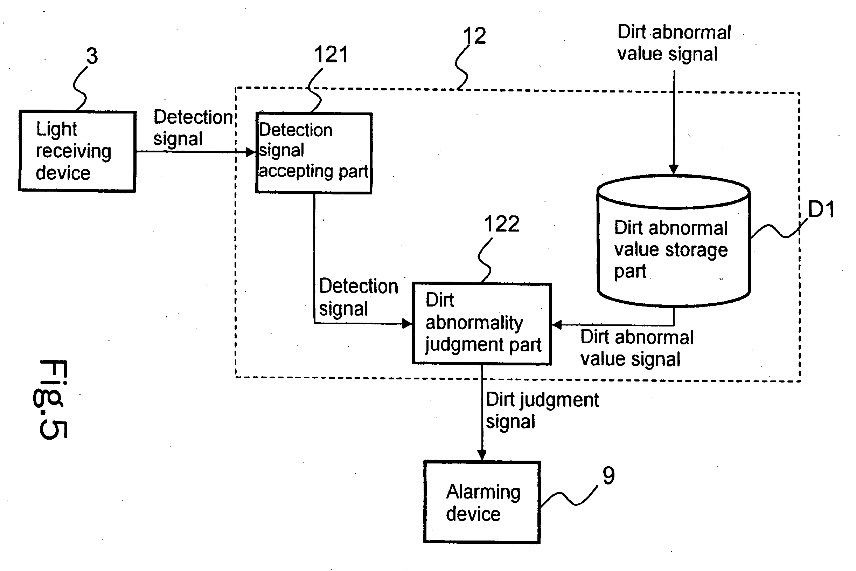

Further, the controller 12 of the present preferred embodiment receives from the light receiving device 3 a detector output signal proportional to the amount of light received, calculates oil mist concentration, and outputs the oil mist concentration in response to an oil mist output request, and also calculates dirt on the transparent window W and then outputs a dirt alarm. The controller 12 is a general-purpose or special-purpose computer including: as shown in FIG. 4, a CPU 1201; an inner memory 1202; an input and output interface 1203; an AD converter 1204; and so on. The controller 12, as a result of activation of the CPU 1201, its surrounding devices, and the like based on programs stored in a predetermined region of the inner memory 1202, functions as a detection signal accepting part 121, a dirt abnormal value storage part D1, a dirt abnormality judgment part 122, etc., as shown in FIG. 5.

The detection signal accepting part 121 receives a detection signal from the light receiving device 3, and outputs this detection signal to the dirt abnormality judgment part 122.

The dirt abnormal value storage part D1 stores a dirt abnormal value signal, which is stored from outside by the operator or the like. Here, the "dirt abnormal value signal" refers to a signal for setting a lowest limit of a decrease in a zero point indicated by a zero point detection signal. Moreover, the "zero point detection signal" refers to a detection signal generated by the background scattering light BLS received by the light receiving device 3 when the irradiated light LB is irradiated by the light emitting device 2 under the absence of oil mist in the oil mist introduction chamber 66, that is, when the diesel engine is stopped. More specifically, the amount of light when the amount of background scattering light BLS decreases by, for example, 20 to 50% or more at a zero point is set and stored as a transparent window dirt abnormal value (hereinafter simply referred to as "dirt abnormal value").

The dirt abnormality judgment part 122 acquires the detection signal from the detection signal accepting part 121, also acquires the dirt abnormal value signal from the dirt abnormal value storage part D1, and compares values of these signals. If the detection signal value (measured value) indicated by the detection signal is smaller than the dirt abnormal value indicated by the dirt abnormal value signal, the dirt abnormality judgment part 122 judges that the transparent window W is dirty to a predetermined degree or more, and outputs a signal of this judgment to an alarm device 9.

The alarm device 9 acquires the judgment signal from the dirt abnormality judgment part 122 of the controller 12 and alarm to or give an alarm to the operator or the like that the transparent window W is dirty to a predetermined degree or more (dirt abnormality). Possible examples of such the alarm device 9 include those which provide a display on the screen, those which emit light, those which generate sound, and the like. Note that a lamp (not shown) provided in the controller 12 may be used as the alarm device 9.

FIG. 4 shows:
1. Light receiving device
2. AD converter
3. Inner memory
4. Input-output interface
5. Oil mist concentration output request
6. Oil mist concentration output
7. Dirt alarm output

FIG. 5 shows:
1. Light receiving device
2. Detection signal
3. Detection signal accepting part
4. Dirt abnormal value signal
5. Dirt abnormality judgment part
6. Dirt abnormal value storage part
7. Dirt judgment signal
8. Alarming device

### <Operation Method>

Next, an operation of the oil mist detector 1 of the present preferred embodiment configured as described above will be described.

### <Dirt Abnormal Value Setting Process>

The dirt abnormal value is basically set at the time of product manufacture, for example, upon zero point adjustment. Specifically, when the light emitting device 2 emits the irradiated light LB under the absence of oil mist, based on the detection signal outputted by the background scattering light BLS received by the light receiving device, an oil mist concentration in this condition is set at 0 mg/L, and for example, 50 to 80% of the detection signal in this condition is set as a dirt abnormal value. When the diesel engine is stopped, that is, under the absence of oil mist, the operator may set these values.

### <Oil Mist Detection Process>

The dirt on the window is detected under the absence of oil mist (when the diesel engine is stopped). In particular, before the diesel engine is restarted after being stopped for a long period of time, a dirt abnormal value previously set by the operator and a measured value (zero point) indicated by the current detection signal are compared with each other to judge whether or not the transparent window W is dirty to a predetermined degree or more. At this point in time, the detection signal accepting part 121 accepts a detection signal from the light receiving device 3, and outputs this detection signal to the dirt abnormality judgment part 122. Then the dirt abnormality judgment part 122 that has received this detection signal compares a measured value indicated by the detection signal and a lower limit of the amount of a decrease in the zero point (dirt abnormal value). If the dirt abnormality judgment part 122 judges that the measured value is below the dirt abnormal value, the dirt abnormality judgment part 122 outputs a signal of this judgment to the alarm device 9. The alarm device 9 that has received this judgment signal alarms this to the operator. This permits notifying the operator that the transparent window is dirty to a predetermined degree or more.

When the diesel engine is in operation (under the presence of oil mist), a normal oil mist detection is performed. Also in this condition, the detection signal accepting part 121 accepts a detection signal from the light receiving device 3 and outputs this detection signal to the dirt abnormality judgment part 122. Then the dirt abnormality judgment part 122 that has received this detection signal compares a measured value and a dirt abnormal value upon oil mist measurement, as is the case with the above. If the dirt abnormality judgment part 122 judges that the measured value is below the dirt abnormal value, the dirt abnormality judgment part 122 outputs a signal of this judgment to the alarm device 9. The alarm device 9 that has received this judgment signal alarms this to the operator. The alarm device 9 may only perform alarming so that measurements can be made continuously thereafter, or a controller 5 may have a function of interrupting the measurement.

On the other hand, if the measured value is not below the dirt abnormal value, the dirt abnormality judgment part 122 does not output the judgment signal to the alarm device 9, whereby measurement is continued without any change.

### <Advantages of the Invention>

With the oil mist detector 1 according to the present preferred embodiment configured as described above, the zero point can be intentionally increased by a zero point shifting structure to detect a decrease in the amount of light received due to dirt abnormality on the transparent window W. This permits simply and reliably detecting dirt on the transparent window W.

Then dirt on the transparent window W caused by long-term oil mist drying and adhesion can be easily and reliably detected, which permits alarming this to the operator before measurement becomes impossible.

Moreover, a measured value and a dirt abnormal value under the presence of oil mist are always compared with each other, thus permitting alarming before remarkable deterioration in the sensitivity of the oil mist detector 1 occurs.

Further, the oil mist detector 1 of the present preferred embodiment so provided as to be directly inserted in the diesel engine has difficulties in zero gas feeding or the like, and cannot perform normal zero calibration employed by an analyzer or the like. However, according to the present preferred embodiment, when the amount of oil mist is small at, for example, the time of engine stop, dirt on the transparent window W can be detected without zero gas feeding. That is, the present preferred embodiment provides an advantage that dirt on the window can be detected at the time of engine stop at which dirt is likely to adhere thereto. Moreover, also in a periodic inspection, it is needless to say that dirt on the window can be instantly detected by just activating a sensor part after removing it from the engine crankcase without any zero gas feeding.

### <Other Modified Preferred Embodiment>

The present invention is not limited to the preferred embodiment described above. In the following description, members corresponding to those in the preferred embodiment described above are provided with the same numerals.

For example, in the preferred embodiment described above, the amount of light blocked is controlled by changing the shapes of the second light blocking member 7 and the third light blocking member 8, and the zero point shifting structure is formed which provides a predetermined detection signal output even under the absence of oil mist. However, in the present preferred embodiment, any other member dedicated for generating the background scattering light BLS may be provided inside the casing 6.

Alternatively, either of the second light blocking member 7 and the third light blocking member 8 may be used as the zero point shifting structure.

Moreover, in the preferred embodiment described above, the slit 8a provided in the third light blocking member 8 is formed allowing for prevention of the generation of the scattering light LS from the irradiated light LB directly hitting the light blocking member 8. Alternatively, the slit 8a may be configured so that the scattering light LS is generated from the irradiated light LB directly hitting the third light blocking member 8.

Further, the inner surface of the leading end part of the second casing part 6B is a tapered surface such that its sectional area becomes gradually smaller toward the leading end, although it may not be formed tapered. Forming this surface not tapered also permits the surface to function as a zero point shifting structure.

In addition, in the preferred embodiment described above, the casing 6 has a single-pipe structure. Alternatively, the casing 6 may have a dual-pipe structure including an inner wall and an outer wall so that the oil mist introduction chamber 66 is connected to the inside of the crankcase C externally fitted on a non-linear path through a through hole in the inner wall, gap, and a through hole in the outer wall. This permits preventing an oil splash (oil droplets) from entering into the oil mist introduction chamber 66. Moreover, introduction and diffusion of oil mist inside are not at all disturbed, which permits proper detection of the oil mist by eliminating the influence of oil splash. Furthermore, only the inner wall and the outer wall may be provided, which permits simple structure and downsizing.

The shape, material, and the like of the casing 6 are not limited to those described in the embodiment above.

Moreover, in the preferred embodiment described above, a dirt abnormal value is inputted by a manufacturer or operator. Alternatively, this value may be transmitted from another computer, or the controller may be provided with a dirt abnormal value setting part. This dirt abnormal value setting part accepts a zero point detection signal from the light receiving device 3 and sets a lowest limit of the amount of a decrease in the zero point indicated by the zero point detection signal.

At this point in time, in the dirt abnormal value setting process, a dirt abnormal value can be set in the following manner. Under the absence of oil mist, the light emitting device 2 emits irradiated light LB. As a result, the light receiving device 3 receives a given amount of background scattering light BLS by the zero point shifting structure. The detection signal accepting part 121 outputs a detection signal in this condition as a zero point detection signal to the dirt abnormal value setting part. Then the dirt abnormal value setting part sets a dirt abnormal value such that it corresponds to, for example, 50 to 80% of the amount of shift in the zero point indicated by the zero point detection signal acquired, and then this dirt abnormal value signal is stored into the dirt abnormal value storage part D1.

## Claims

1. An oil mist detector (1) adapted to be arranged in a crankcase (C) of an internal combustion engine comprising:
a casing (6) having an oil mist introduction chamber (66); and
a transparent window (W) provided in the oil mist introduction chamber (66);
a light emitting device (2) and a light receiving device (3) so arranged as to face an outer side of the transparent window (W) provided in the oil mist introduction chamber (66);
wherein the light emitting device (2) is arranged for irradiating light to a predetermined detection region (S) in the oil mist introduction chamber (66) through the transparent window (W) and
the light receiving device (3) is arranged for receiving through the transparent window (W) scattering light generated from the irradiated light hitting oil mist present in the detection region (S), thereby achieving oil mist detection,
**characterized in that** the oil mist detector (1) further comprises
a zero point shifting structure (7, 8) provided for making a given amount of background scattering light from the casing (6) incident on the light receiving device (3) under absence of oil mist in the oil mist introduction chamber (66),
and
a controller (12) for accepting a detection signal from the light receiving device (3) and performing predetermined calculation processing, wherein the controller (12) comprises a dirt abnormality judgment part (122) which is adapted to receive a zero point signal as a detection signal detecting only background scattering light, and
adapted to compare a detection signal value indicated by a detection signal from the light receiving device (3) and a dirt abnormal value being a preset value by which the zero point is allowed to be decreased,
and adapted to judge that the transparent window (W) is dirty if the detection signal value is smaller than the dirt abnormal value.

2. The oil mist detector (1) according to claim 1,
wherein the casing (6) forms a cylindrical shape,
wherein the zero point shifting structure is formed of light blocking members (7, 8), and wherein the zero point shifting structure is formed by shortening a length of one of the light blocking members (7) to a length enabling a shift of a zero point of a detection signal by making a given amount of background scattering light (BLS) from a inner wall of the casing (6) incident on the light receiving device (3), the light blocking member (7) being provided so as to partition an optical axis (6L) extended line of the irradiated light (LB) traveling from the transparent window (W) toward the detection region (S) and an optical axis extended line of the scattering light (LS) traveling from the detection region (S) toward the transparent window (W), the decrease in the zero point being caused by dirt on the transparent window (W).

3. The oil mist detector (1) according to claim 1, further comprising an alarm device (9) which receives a judgment signal from the dirt abnormality judgment part (122) and alarming the dirt on the transparent window (W).

4. The oil mist detector (1) according to any of claims 1 to 3, comprising
a first refraction device (41) being arranged between the light emitting device (2) and the transparent window (W) for refracting the light emitted from the light emitting device (2) to the transparent window (W); and
a second refraction device (42) being arranged between the light receiving device (3) and the transparent window (W) for refracting the light coming from the oil mist introduction chamber (66) and passing through the window (W) to the light receiving device (3);
wherein the first refraction device (41) and the second refraction device (42) are separated by a light blocking member (43).

5. The oil mist detector (1) according to any of claims 1 to 4, wherein the casing (6) has a
first casing part (6A) housing the light emitting device (2) and the light receiving device (3) and a
second casing part (6B) housing the oil mist introduction chamber (66).

6. The oil mist detector (1) according to claim 5 as dependent on claim 4, wherein the zero point shifting structure (7,8) comprises a light blocking member (7) being arranged in the front part of the second casing part (6B) on a side opposite to the refraction devices (41, 42) and extending between the front of the detection region (S) and the inner surface of the leading end of the second casing part (6B) for forming two spaces (661, 662) by halving the leading end side of the second casing part (6B).

7. The oil mist detector (1) according to claims 5 or 6, wherein the zero point shifting structure (7,8) comprises a light blocking member (8)
being provided orthogonally to a main axis (6L) of the casing (6) and
being arranged between the transparent window (W) and the oil mist introduction chamber (66).

## Patentansprüche

1. Ölnebeldetektor (1), dazu geeignet in einem Motorgehäuse (C) eines Verbrennungsmotors angeordnet zu sein, mit:
einem Gehäuse (6) mit einer Ölnebeleinbringkammer (66); und
einem in der Ölnebeleinbringkammer (66) angeordneten transparenten Fenster (W);
einer Lichtemissionsvorrichtung (2) und einer Lichtempfangsvorrichtung (3), die derart angeordnet sind, dass sie einer äußeren Seite des in der Ölnebeleinbringkammer (66) angeordneten transparenten Fensters (W) gegenüberliegen; wobei
die Lichtemissionsvorrichtung (2) angeordnet ist, um durch das transparente Fenster (W) Licht auf einen vorgegebenen Detektionsbereich (S) in der Ölnebeleinbringkammer (66) auszustrahlen und
die Lichtempfangsvorrichtung (3) angeordnet ist, durch das transparente Fenster (W) gestreutes Licht zu empfangen, das durch das ausgestrahlte Licht erzeugt wird, das auf Ölnebel trifft, der in dem Detektionsbereich (S) vorhanden ist, und dadurch die Ölnebeldetektion erreicht,
**dadurch gekennzeichnet, dass** der Ölnebeldetektor (1) des Weiteren aufweist:
eine Nullpunktverschiebungsstruktur (7,8), die dazu angeordnet ist unter Abwesenheit von Ölnebel in der Ölnebeleinbringkammer (66) einen bestimmten Betrag von Hintergrundstreulicht von dem Gehäuse (6) auf die Lichtempfangsvorrichtung (3) einfallen zu lassen; und
einer Steuerung (12) zum Annehmen eines Detektionssignals von der Lichtempfangsvorrichtung (3) und zum Ausführen von vorgegebenen Rechenschritten, wobei die Steuerung (12) ein Schmutzabweichungsbeurteilungselement (122) aufweist, das geeignet ist, ein Nullpunktsignal als Detektionssignal zu empfangen, das nur Hintergrundstreulicht detektiert, und
das geeignet ist, einen Detektionssignalwert, der durch ein Detektionssignal von der Lichtempfangseinheit (3) angegeben wird, und einen Schmutzabweichwert zu vergleichen, der ein vorgegebener Wert ist, durch den erlaubt wird, den Nullpunkt zu verringern, und
das geeignet ist, festzustellen, dass das transparente Fenster (W) schmutzig ist, wenn der Detektionssignalwert kleiner als der Schmutzabweichwert ist.

2. Ölnebeldetektor (1) gemäß Anspruch 1,
wobei das Gehäuse (6) eine zylindrische Form ausbildet,
wobei die Nullpunktverschiebungsstruktur aus Lichtblockiereinrichtungen (7,8) ausgebildet ist, und
wobei die Nullpunktverschiebungsstruktur durch Verkürzen einer Länge einer der Lichtblockiereinrichtungen (7) auf eine Länge ausgebildet ist, die es erlaubt, einen Nullpunkt eines Detektionssignals dadurch zu verschieben, einen bestimmten Betrag von Hintergrundstreulicht (BLS) von einer inneren Wand des Gehäuse (6) auf die Lichtempfangsvorrichtung (3) einfallen zu lassen, wobei die Lichtblockiereinrichtung (7) derart angeordnet ist, dass sie eine verlängerte Linie der optische Achse (6L) des von dem transparenten Fenster (W) auf den Detektionsbereich (S) zu laufenden ausgestrahlten Lichts (LB) und eine verlängerte Linie der optischen Achse des Streulichts (LS) teilt, das von dem Detektionsbereich (S) auf das transparente Fenster (W) zu läuft, wobei die Verringerung des Nullpunktes durch Schmutz auf dem transparenten Fenster (W) verursacht ist.

3. Ölnebeldetektor (1) gemäß Anspruch 1, des Weiteren mit einer Warnvorrichtung (9), die ein Beurteilungssignal von dem Schmutzabweichungsbeurteilungselement (122) empfängt und vor Schmutz auf dem transparenten Fenster (W) warnt.

4. Ölnebeldetektor (1) gemäß einem der Ansprüche 1 bis 3 mit
einer ersten Brechungsvorrichtung (41), die zum Brechen des von der Lichtemissionsvorrichtung (2) auf das transparente Fenster (W) hin emittierten Lichts zwischen der Lichtemissionsvorrichtung (2) und dem transparenten Fenster (W) angeordnet ist; und
einer zweiten Brechungsvorrichtung (42), die zum Brechen des von der Ölnebeleinbringkammer (66) kommenden und durch das transparente Fenster (W) durchlaufenden Lichts zwischen der Lichtempfangsvorrichtung (3) und dem transparenten Fenster (W) angeordnet ist;
wobei die erste Brechungsvorrichtung (41) und die zweite Brechungsvorrichtung (42) durch eine Lichtblockiereinrichtung (43) getrennt sind.

5. Ölnebeldetektor (1) gemäß einem der Ansprüche 1 bis 4, wobei das Gehäuse (6)
einen ersten Gehäuseteil (6A), der die Lichtemissionsvorrichtung (2) und die Lichtempfangsvorrichtung (3) aufnimmt und
einen zweiten Gehäuseteil (6B) aufweist, der die Ölnebeleinbringkammer (66) aufnimmt.

6. Ölnebeldetektor (1) gemäß dem von Anspruch 4 abhängigen Anspruch 5, wobei die Nullpunktverschiebungsstruktur (7, 8) eine Lichtblockiereinrichtung (7) aufweist, die in dem vorderen Teil des zweiten Gehäuseteils (6B) auf einer den Brechungsvorrichtungen (41, 42) gegenüberliegenden Seite angeordnet ist und sich zwischen der Vorderseite des Detektionsbereichs (S) und der inneren Oberfläche des vorderen Endes des zweiten Gehäuseteils (6B) erstreckt, um zwei Räume (661, 662) durch halbieren der vorderen Endseite des zweiten Gehäuseteils (6B) auszubilden.

7. Ölnebeldetektor (1) gemäß Anspruch 5 oder 6, wobei die Nullpunktverschiebungsstruktur (7, 8) eine Lichtblockiereinrichtung (8) aufweist,
die orthogonal zu einer Hauptachse (6L) des Gehäuses (6) und
zwischen dem transparenten Fenster (W) und der Ölnebeleinbringkammer (66) angeordnet ist.

## Revendications

1. Détecteur de brouillard d'huile (1) apte à être disposé dans un carter (C) d'un moteur à combustion interne, comprenant :
un boîtier (6) comportant une chambre d'introduction de nuage d'huile (66) ; et
une fenêtre transparente (W) prévue dans la chambre d'introduction de brouillard d'huile (66) ;
un dispositif émetteur de lumière (2) et un dispositif récepteur de lumière (3) qui sont disposés de manière à faire face à un côté extérieur de la fenêtre transparente (W) prévue dans la chambre d'introduction de brouillard (66) ;
étant précisé que le dispositif émetteur de lumière (2) est conçu pour propager de la lumière vers une zone de détection prédéterminée (S), dans la chambre d'introduction de brouillard d'huile (66), à travers la fenêtre transparente (W), et
que le dispositif récepteur de lumière (3) est conçu pour recevoir à travers la fenêtre transparente (W) la lumière diffusée produite à partir de la lumière propagée qui atteint le brouillard d'huile présent dans la zone de détection (S), réalisant ainsi la détection de brouillard d'huile,
**caractérisé en ce que** le détecteur de brouillard d'huile (1) comprend également
une structure de décalage de point zéro (7, 8) prévue pour rendre une quantité donnée de lumière diffusée de fond provenant du boîtier (6) incidente sur le dispositif récepteur de lumière (3) en l'absence de brouillard d'huile dans la chambre d'introduction de brouillard d'huile (66),
et un dispositif de commande (12) pour accepter un signal de détection provenant du dispositif récepteur de lumière (3) et pour effectuer une opération de calcul prédéterminée, étant précisé que le dispositif de commande (12) comprend une partie de jugement d'anomalie de saleté (122) qui est apte à recevoir un signal de point zéro comme signal de détection détectant seulement une lumière diffusée de fond, et
qui est apte à comparer une valeur de signal de détection indiquée par un signal de détection provenant du dispositif récepteur de lumière (3), et une valeur anormale de saleté qui est une valeur préréglée par laquelle le point zéro est autorisé à être réduit,
et qui est apte à juger que la fenêtre transparente (W) est sale si la valeur de signal de détection est inférieure à la valeur anormale de saleté.

2. Détecteur de brouillard d'huile (1) selon la revendication 1,
étant précisé que le boîtier (6) définit une forme cylindrique,
que la structure de décalage de point zéro se compose d'éléments de blocage de lumière (7, 8),
et que la structure de décalage de point zéro est formée grâce au raccourcissement d'une longueur de l'un des éléments de blocage de lumière (7) à une longueur permettant un décalage d'un point zéro d'un signal de détection en rendant une quantité donnée de lumière diffusée de fond (BLS) provenant d'une paroi intérieure du boîtier (6) incidente sur le dispositif récepteur de lumière (3), l'élément de blocage de lumière (7) étant disposé de manière à séparer une ligne, prolongeant l'axe optique (6L), de la lumière propagée (LB) qui va de la fenêtre transparente (W) vers la zone de détection (S), et une ligne, prolongeant l'axe optique, de la lumière diffusée (LS) qui va de la zone de détection (S) vers la fenêtre transparente (W), la diminution du point zéro étant causée par la saleté qui se trouve sur la fenêtre transparente (W).

3. Détecteur de brouillard d'huile (1) selon la revendication 1, comprenant également un dispositif d'alarme (9) qui reçoit un signal de jugement de la partie de jugement d'anomalie de saleté (122), et signalant la saleté sur la fenêtre transparente (W).

4. Détecteur de brouillard d'huile (1) selon l'une quelconque des revendications 1 à 3, comprenant
un premier dispositif de réfraction (41) qui est disposé entre le dispositif émetteur de lumière (2) et la fenêtre transparente (W) pour réfracter la lumière émise à partir du dispositif émetteur de lumière (2) vers la fenêtre transparente (W) ; et
un second dispositif de réfraction (42) qui est disposé entre le dispositif récepteur de lumière (3) et la fenêtre transparente (W) pour réfracter la lumière provenant de la chambre d'introduction de brouillard d'huile (66) et traversant la fenêtre (W) vers le dispositif récepteur de lumière (3) ;
étant précisé que le premier dispositif de réfraction (41) et le second dispositif de réfraction (42) sont séparés par un élément de blocage de lumière (43).

5. Détecteur de brouillard d'huile (1) selon l'une quelconque des revendications 1 à 4, étant précisé que le boîtier (6) comporte
une première partie de boîtier (6A) dans laquelle sont logés le dispositif émetteur de lumière (2) et le dispositif récepteur de lumière (3), et
une seconde partie de boîtier (6B) dans laquelle est logée la chambre d'introduction de brouillard d'huile (66).

6. Détecteur de brouillard d'huile (1) selon la revendication 5 lorsqu'elle est dépendante de la revendication 4, étant précisé que la structure de décalage de point zéro (7, 8) comprend un élément de blocage de lumière (7) qui est disposé dans la partie avant de la seconde partie de boîtier (6B), sur un côté opposé aux dispositifs de réfraction (41, 42), et qui s'étend entre l'avant de la zone de détection (S) et la surface intérieure de l'extrémité avant de la seconde partie de boîtier (6B) pour former deux espaces (661, 662) en divisant en deux le côté d'extrémité avant de ladite seconde partie de boîtier (6B).

7. Détecteur de brouillard d'huile (1) selon les revendications 5 ou 6, étant précisé que la structure de décalage de point zéro (7, 8) comprend un élément de blocage de lumière (8)
qui est orthogonal par rapport à un axe principal (6L) du boîtier (6) et
qui est disposé entre la fenêtre transparente (W) et la chambre d'introduction de brouillard d'huile (66).
